# EUROPEAN PATENT SPECIFICATION

(11) **EP 4 612 371 B1**
(45) Date of publication and mention of the grant of the patent: **26.08.2026**
(21) Application number: 22835150.8
(22) Date of filing: 31.10.2022
(51) Int. Cl.: D21J 7/00, D21J 3/00, D21J 3/10

(54) **PRESSURE MEMBERS**
DRUCKELEMENTE
ÉLÉMENTS DE PRESSION

(43) Date of publication of application: 10.09.2025
(73) Proprietor: Peridot Print LLC, Palo Alto, CA 94304 (US)
(72) Inventor: LEDESMA FERNANDEZ, Javier, 08174 Sant Cugat del Vallès Barcelona (ES); PARE BARNIOL, Lluis, 08174 Sant Cugat del Vallès Barcelona (ES)
(74) Representative: Haseltine Lake Kempner LLP
(86) International application number: PCT/US2022/048399
(87) International publication number: WO 2024/096854

(56) References cited:
- WO-A1-2012/033449
- WO-A1-2022/220805
- WO-A1-94/24371

## Description

### BACKGROUND

Various types of products may be fabricated from a pulp of material. Particularly, a pulp molding die that includes a forming screen may be immersed in the pulp of material and the material in the pulp may form into the shape of the forming screen. The forming screen may have a desired shape of the product to be formed and may thus have a complex shape in some instances. The forming screen may include numerous pores for liquid passage. During formation of the product, a vacuum force may be applied through the pulp molding die, which may cause the material in the pulp to be sucked onto the forming screen and form into a shape that matches the shape of the pulp molding die. The material may be removed from the forming screen and may be solidified to have the desired shape.

WO9424371A1 describes modular moulding tools for use in a machine for manufacturing shells or thin-walled blanks from pulp material. WO2012033449A1 discloses a mould arrangement including a loose sintered metal mould having a front side and an opposite rear side secured to a base plate, wherein the base plate includes elongated channels including a suction channel for delivering suction pressure to the mould. At least one of the channels includes a heating rod. WO2022220805A1 discloses partitioning a 3D model into separate sections, and modifying a 3D model to model the component as the separate sections.

### SUMMARY OF INVENTION

The invention is set out in the appended claims.

### BRIEF DESCRIPTION OF DRAWINGS

Features of the present invention are illustrated by way of example and are not limited in the following figure(s), in which like numerals indicate like elements, in which:
FIG. 1 shows a schematic diagram illustrating a pressure regulation device including a plurality of pressure lines, according to an example of the present invention;
FIG. 2A shows a side view of a pressure regulation device including an alignment member, according to an example of the present invention;
FIG. 2B shows a side view of a pressure regulation device including a plurality of manifolds and an alignment member, according to an example of the present invention;
FIG. 2C shows a side view of a pressure regulation device including manifolds having different sizes, according to an example of the present invention;
FIG. 3A shows a cross-sectional side view of a pulp molding system including a pressure member and a molded fiber toolset, according to an example of the present invention;
FIG. 3B shows a cross-sectional side view of a pulp molding system including an alignment member in between a molded fiber toolset and a mating element of the pressure member, according to an example of the present invention;
FIG. 3C shows a cross-sectional side view of a pulp molding system including a tool set comprising a forming mold, a forming screen, and a transfer mold, according to an example of the present invention;
FIG. 4 shows a method for pressurizing a volume defined between a forming mold and a mating element of a pressure member, according to an example of the present invention; and
FIG. 5 shows a method for determining a tool underperformance, according to an example of the present invention;

### DETAILED DESCRIPTION

For simplicity and illustrative purposes, the present invention is described by referring mainly to examples. In the following description, numerous specific details are set forth in order to provide a thorough understanding of the present invention. It will be readily apparent, however, that the present invention may be practiced without limitation to these specific details. Throughout the present disclosure, elements referred to with the same terms in different examples should be interpreted as being the same or similar elements. In other instances, some methods and structures have not been described in detail so as not to unnecessarily obscure the present disclosure.

Throughout the present disclosure, the terms "a" and "an" are intended to denote at least one of a particular element. As used herein, the term "includes" means includes but not limited to, the term "including" means including but not limited to. The term "based on" means based at least in part on.

Pulp molding dies may be used to obtain molded fiber articles from a slurry of fibers. In some examples, the wet part may be formed in a toolset that may include a forming screen. Particularly, a first surface of the wet part may be in contact with the forming screen. To form the wet part from fibers in a slurry of the fibers, vacuum pressure may be applied through the forming screen such that the first surface of the wet part matches the shape and contours of the forming screen. Following formation of the wet part on the forming screen, the forming screen and the wet part may be removed from the slurry. In some examples, the toolset may further comprise a transfer screen to contact with a second surface of the wet part. Upon removing the forming screen from the slurry, the transfer screen may be positioned near the second surface. Then, vacuum pressure may be applied through the transfer screen onto the second surface of the wet part to cause the second surface to have the shape and contours of the transfer screen.

In some examples, a pressure system may be used for generating molded fiber parts from a slurry of fibers. In particular, the pressure system may be used to apply a pressure level through an outlet of the pressure system, the outlet being in fluidic communication with apertures of a toolset used for obtaining a wet part. In some examples, to generate the wet part from the slurry of fibers, the pressure system may apply a pressure level associated with a geometry of a molded fiber part to be obtained. In some examples, the wet part may undergo additional operations to obtain the molded fiber part (e.g., dewatering operations and/or drying operations).

As used herein, the term "slurry of fibers" will be used to refer to a mixture including a liquid and fibers. The liquid vehicle of the slurry of fibers may be water or another type of suitable liquid in which fibers may be mixed into the slurry. The fibers in the slurry may include fibers of paper, wood, fibrous crops, bamboo, and/or the like.

When applying vacuum through a mold of a toolset (e.g., the forming mold or the transfer mold), pressure systems may be in fluidic communication with the respective mold. In some examples, a pressure system may be fluidically connected to a volume defined between the mold and a surface of the pressure system for receiving the mold. In an example, a pressure system may comprise a mating element arranged to receive a mold, and the pressure system may adjust a pressure value in a volume defined between the mold and the mating element. However, some molds may comprise non-uniform surfaces (e.g., uneven surfaces, non-symmetrical surfaces, and the like) at different distances from an outlet of the pressure system, and the pressurization of the volume using the pressure system may result in non-uniform pressures across the mold. Also, the size of the mold may be affected by the type of pressure system used in the vacuum operations. For instance, when having a large mold, regions of the mold closer to the outlet of the pressure system may receive different pressure levels with respect to regions located farther away from the outlet, thereby leading to an uneven pressure distribution across the surface of the mold facing the outlet of the pressure system.

Among others, non-uniform pressures across the forming molds and transfer molds may result in part defects in the molded fiber parts obtained from slurry of fibers. For instance, non-uniform pressures during the generation of a wet part may result in different thicknesses or non-uniform mechanical properties (e.g., density, porosity of the fibers, grammage, concentration of the fibers, and the like) across the molded fiber part obtained from the wet part. In other examples, non-uniform pressure levels across the mold may result in a portion of the wet part being stuck onto a component of the toolset (e.g., a forming screen associated with the forming mold or the transfer screen associated with the transfer mold), a portion of the wet part being torn, presence of cracks in the molded fiber part, among others.

Disclosed herein are examples of pressure regulation devices, pulp molding systems, and methods to reduce the negative effects associated to the application of non-uniform pressures across a mold.

As used herein, the term "mold" will be used to refer to forming molds and transfer molds. In some examples, a forming mold may be associated with a respective forming screen. Similarly, in some examples, a transfer mold may be associated with a transfer screen. Also, as used herein, the term "molded fiber toolset" will be used to refer to a plurality of components used for generating a final part from a slurry of fibers. In some examples, the toolset may comprise a forming mold and a forming screen. In yet other examples, the toolset may further comprise a transfer mold. In some other examples, the toolset may further comprise a transfer mold and a transfer screen.

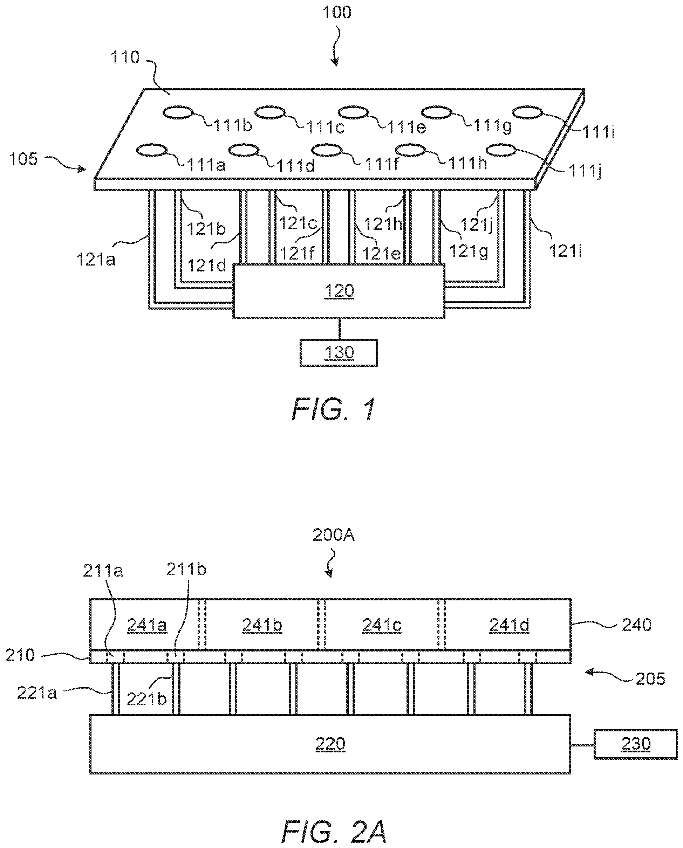

Referring now to FIG. 1, a pressure regulation device 100 is shown. The pressure regulation device 100 may be used, for instance, to generate a wet part from a slurry of fibers. The pressure regulation device 100 comprises a pressure member 105 and a controller 130. The pressure member 105 includes a mating element 110 comprising a plurality of outlets 111a-111j and a pressure source 120, being the pressure source 120 fluidically connected to the plurality of outlets 111a-111j of the mating element 110 via a respective plurality of pressure lines 121a-121j. Examples of pressure sources 120 include vacuum devices to apply a vacuum pressure (e.g., vacuum pump), vacuum devices including electronic valves, and arrays of valves controllable by a controller to separately pressurize pressure lines fluidically connected to a vacuum device. In some examples, valves part of a pressure source may be selectively opened (or closed) to enable (or block) fluidic channels.

A surface of the mating element 110 (e.g., illustrated top surface) of the pressure regulation device 100 is to receive a forming mold or a transfer mold such that a volume defined between the mold and the mating element 110 is fluidically connected to the plurality of outlets in the mating element 110. The plurality of outlets in the mating element 110 comprises outlets 111a-111j, each of the outlets 111a-111j being associated with a respective pressure line of pressure lines 121a- 121j. The controller 130 of the pressure regulation device 100 is to control the pressure source 120 to separately pressurize the pressure lines 121a-121j. By separately pressurizing the pressure lines 121a-121j, the pressure differentials across a surface of the mold that faces the mating element 110 are reduced.

As previously described, when applying a pressure level via a pressure system, the relative distances from the outlet of the pressure system to the mold may result in a non-uniform pressure distribution across the surface of the mold. In the pressure regulation device 100, the controller 130 is to control the pressure source 120 to separately pressurize the pressure lines 121a-121j such that variability of the pressure levels (e.g., pressure differentials) across the mold is reduced. As used herein, the term "separately pressurize" is used to refer to individually set the pressure lines 121a-121j at a respective pressure value, being the respective pressure value a common pressure value across the pressure lines, a different pressure value across the pressure lines, or a pressure value for a respective pressure line based on a geometry of the mold and/or an outlet arrangement across the mating element.

As used herein, the term "controller" will be used to refer to any combination of hardware and programming to implement the functionalities described herein. In some examples, such combinations of hardware and programming may be implemented in a number of different ways. For example, the programming of modules may be processor-executable instructions stored on at least one non-transitory machine-readable storage medium and the hardware for modules may include at least one processor to execute those instructions. In some other examples, multiple modules may be collectively implemented by a combination of hardware and programming, as described above. In some other examples, the functionalities of the controller may be, at least partially, implemented in the form of electronic circuitry.

In some examples, the controller 130 is to control the pressure source 120 to apply a common pressure level to the pressure lines 121a-121}. In other examples, the controller 130 is to control the pressure source 120 to selectively pressurize each, or a subset, of the pressure lines 121a-121j based on a geometry of the mold and/or a geometry of a pulp part (e.g., a molded fiber part) to be generated. In some other examples, the pressure levels for the pressure lines may be based on a distribution of the outlets across the mating element 110 of the pressure member 105. Accordingly, the controller 130 may control the pressure source 120 to selectively pressurize the pressure lines 121a-121j based on a geometry of the mold and an outlet arrangement of the outlets 111a-111j across the mating element 110. In some other examples, the controller 130 may control the pressure source 120 to selectively pressurize the pressure lines 121a-121j based on a geometry of the mold, a geometry of a pulp part to be obtained, and an outlet arrangement of the outlets 111a-111j across the mating element 110. As used herein, the term "selectively pressurize" is used to refer to individually set each of the pressure lines 121a-121j at a respective pressure value in view of at least one of a geometry of the mold, and an outlet arrangement across the mating element. In some examples, the controller 130 may control the pressure source 120 to pressurize the pressure lines at pressure values within a predetermined range.

Although the mating element 110 of the illustrated pressure regulation device 100 includes ten outlets, it should be noted that other examples of mating elements may comprise additional or fewer outlets. However, to reduce the negative effects resulting from non-uniform pressures across the mold, the number of outlets in the mating element may be two or more outlets to separately (or, in some examples, selectively) pressurize a volume defined between the mold and the mating element 110 of the pressure regulation device 100. Similarly, in some examples, the plurality of outlets of the mating element 110 is uniformly distributed throughout the mating element 110 in an outlet arrangement in which each of the outlets is at a common distance from an adjacent outlet. However, in other examples, the outlet arrangement may be non-uniform, or may be in the form of other geometrical shapes (e.g., outlets concentrically arranged with respect to a center of the mating element 110 or outlets arranged in accordance with a geometry of a mold to be received).

Referring now to FIG. 2A, a side view of a pressure regulation device 200A including an alignment member 240 is shown. The pressure regulation device 200A comprises a pressure member 205 and a controller 230. The pressure member 205 comprises a mating element 210 including a plurality of outlets 211a-211b and a pressure source 220 fluidically connected to the outlets of the mating element 210 via respective pressure lines 221a-221b. As previously explained in reference to the pressure regulation device 100, the controller 230 is to control the pressure source 220 to separately pressurize the pressure lines 221a-221b.

The alignment member 240 of the pressure regulation device 200A comprises a plurality of apertures 241a-241d therethrough. For illustrative purposes, the location and distribution of the vertical walls of the alignment member 240 has been represented using dashed line. The plurality of apertures 241a-241d of the alignment member 240 comprises a first aperture 241a, a second aperture 241b, a third aperture 241c, and a fourth aperture 241d. In FIG. 2A, the mating element 210 receives the alignment member 240 such that a further volume defined by a mold received by the pressure member 205 and the alignment member 240 is fluidically connected to the plurality of outlets via apertures of the alignment member 240. The apertures 241a-241d of the alignment member 240 enable fluid pathways for adjusting pressure in the further volume defined between a mold received by the mating element 210 and the mating element 210. As used herein, the term "volume" will be used to refer to a volume defined between the mold and the mating element and the term "further volume" will be used to refer to an additional volume defined between the mold and the alignment element.

In the example represented in FIG. 2A, the pressure regulation device 200A includes multiple outlets of the mating element 210 fluidically connected to the same aperture of the alignment member. As a result, the first aperture 241a of the alignment member 240 is fluidically connected to a first outlet 211a and a second outlet 211b of the plurality of outlets, each of the outlets 211a-211b being connected to the pressure source 220 via a respective pressure line of the pressure lines 221a-221b. However, alternative examples may include other configurations without departing from the scope of the present disclosure, such as a different number of outlets of the mating element 210 being connected to an aperture of the alignment member 240.

In some examples the alignment member 240 may be a lattice frame comprising the plurality of apertures uniformly distributed throughout the lattice frame. As used herein, the term "lattice" should be understood as an arrangement of a member or members (e.g., branches, beams, joists, columns, posts, rods, fins, etc.). The member or members of a lattice structure may intersect at a diagonal, perpendicular, or oblique (e.g., non-perpendicular and non-parallel) angle.

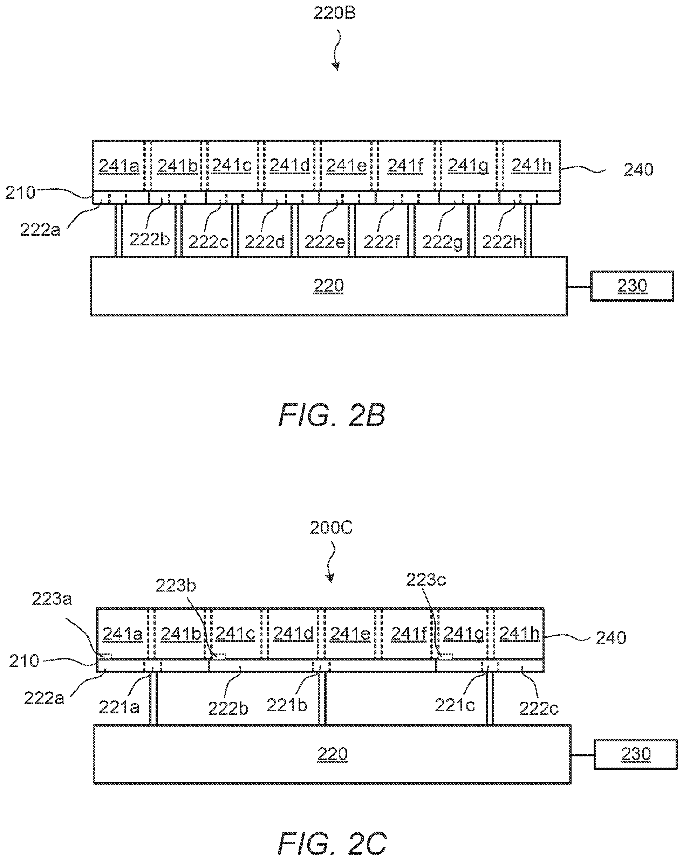

Referring now to FIG. 2B, a side view of a pressure regulation device 200B is shown. Elements of the pressure regulation device 200B previously described in reference to the pressure regulation device 200A of FIG. 2A have been referenced using the same reference numerals. In the pressure regulation device 200B, an alignment member 240 comprises a plurality of apertures 241a-241h and a mating element 210 comprises a plurality of manifolds 222a-222h. As used herein, the term "manifold" refers to a surface including at least one aperture, the surface being arranged to engage with a mold (or an alignment element, if any). In some examples, multiple manifolds may be used for engaging with the same mold (or the same alignment element 240, if any).

In the pressure regulation device 200B, each of the manifolds 222a-222h comprises a respective outlet and is to engage with one aperture of the plurality of apertures 241a-241h. By selectively connecting each of the manifolds 222a-222h to a respective aperture 241a-241h of the alignment member 240, a user of the pressure regulation device 200B may modify a manifold layout based on the shape of the mold to be pressurized using the pressure regulation device 200B. As used herein, the term "manifold layout" refers to a spatial arrangement of the manifolds with respect to each other. In some examples, a manifold layout may include a dummy manifold which serves to prevent pressure leaks by sealing an open space though a volume defined between the mold and the mating element 210 and the external environment. Accordingly, the use of dummy manifolds prevents pressure leaks by sealing open spaces between the volume defined between the mold (not shown) and the plurality of manifolds 222a-222h and the external environment.

In some examples, to removably attach the manifolds 222a-222h to the alignment member 240, each manifold may comprise a connector having a complementary shape to engage with an aperture of the alignment member 240.

Although the apertures 241a-241h extend in a vertical direction with respect to the mating element 210, it should be understood that alternative configurations may be possible. Similarly, alternative cross-section shapes for the apertures 241a-241h may be possible, such as a polygonal cross-section including but not limited to triangular cross-sections, circular cross-sections, rectangular cross-sections, pentagonal cross-sections, hexagonal cross-sections, among others.

According to some examples, a pressure regulation device may comprise an alignment member and a mating element including manifolds of different sizes. Each of the manifolds may be removably attached to at least one aperture of the alignment member in accordance with a manifold layout. In some examples, a manifold may be arranged to engage with at least two adjacent apertures of the alignment member. In this fashion, a manifold including at least one outlet may be used for adjusting a pressure value in the volume defined between the mold and the mating element via adjacent apertures of the alignment member. In some examples, the manifold layout may be set based on a geometry of the mold to be received by the mating element via the alignment member. Accordingly, the manifold layout may be modified in view of the geometry of the mold to be received.

Referring now to FIG. 2C, a side view of a pressure regulation device 200C including manifolds of different sizes is shown. Elements of the pressure regulation device 200C previously described in reference to the pressure regulation devices 200A and 200B of FIGs. 2A and 2B have been referenced using the same reference numerals. In the pressure regulation device 200C, a mating element 210 incudes a first manifold 222a, a second manifold 222b, and a third manifold 222c. Each of the manifolds 222a-222c comprises a respective outlet 211a-211c in fluidic communication with a pressure source 220 of the pressure regulation device 200C via a respective pressure line. In some examples, the manifolds 222a-222c may be removable from their respective pressure lines, thereby enabling to replace a manifold of a first size with a different manifold having a different size. As a result, the flexibility of the pressure regulation device 200C is increased compared to mating elements comprising manifolds which are not removable from the pressure lines.

As previously described the alignment member 240 may be arranged to receive a mold such that a further volume defined between the received mold and the alignment member 240 is fluidically connected to the pressure source 220 via outlets 211a-211c. The pressure regulation device 200C further comprises a plurality of sensors 223a-223c to measure pressures in regions of a volume defined between the received mold and the mating element 210. Over generation of a wet part from a slurry of fibers and/or during transfer of the wet part, the pressure measurements made by the plurality of sensors 223a-223c may be used by the controller 230 to determine a tool underperformance of a forming mold or a transfer mold. The plurality of sensors 223a-223c comprises a first pressure sensor 223a, a second pressure sensor 223b, and a third pressure sensor 223c. In some examples, upon the pressure regulation device 200C receiving a mold, the controller 230 may determine a tool underperformance based on the pressure measurements by the plurality of pressure sensors. Examples of tool underperformance comprise tool clogging, pressure leak detection, among others. In some examples, to determine the tool underperformance, the controller 230 may compare the measured pressure to a threshold pressure value representing an expected pressure value when using the pressure source 320 to separately pressurize the pressure lines.

In some examples, at least one manifold of the plurality of manifolds 222a-222c is to engage with at least two adjacent apertures 241a-241h of the alignment member 240. In some other examples, each of the manifolds of the plurality of manifolds 222a-222c may comprise a connector having a complementary shape to engage with an aperture of the alignment member 240. As previously defined, the apertures 241a-241h of the alignment member 240 may comprise different types of polygonal cross-sections.

For simplicity, different examples of pressure regulations devices have been described with reference to FIGs. 1 to 2C. However, it should be noted that alternative examples may be derived without departing from the scope of the present invention. In an example, the pressure regulation devices 100, 200A, and 200B may comprise the plurality of sensors described in reference to the pressure regulation device 200C to determine a tool underperformance during operation. Similarly, in other examples, the pressure regulation devices 100, 200A, and 200B may include manifolds of different sizes, such as the plurality of manifolds previously described in reference to the pressure regulation device 200C. In some other examples, two or more outlets of a mating element of a pressure member may be fluidically connected to a number of pressure lines smaller than the number of outlets. For instance, in reference to the pressure regulation device 100, the outlets 111a-111b may be fluidically connected to a single pressure line instead of being connected to a first pressure line 121a and a second pressure line 121b, respectively. It is further noted that some additional examples may include a combination of the some or all of the above features.

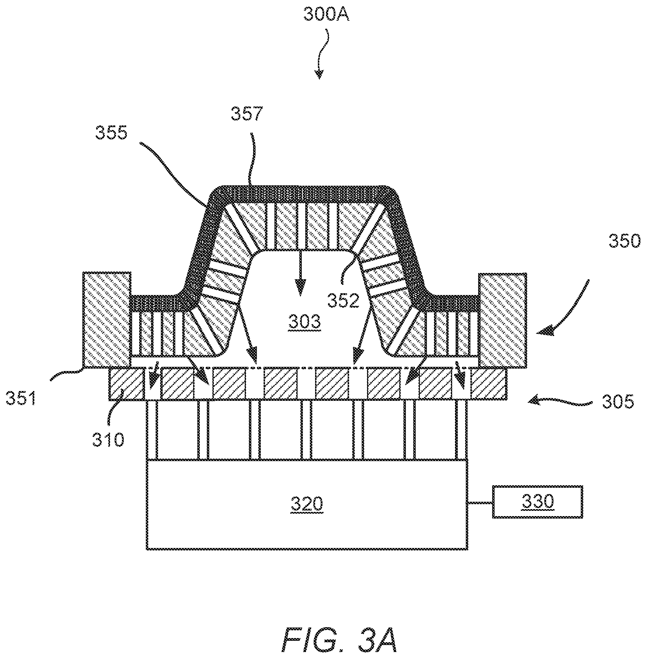

Referring now to FIG. 3A, a cross-sectional view of a pulp molding system 300A including a pressure member 305 and a molded fiber toolset 350 is shown. As previously defined in reference to FIGs. 1 to 2B, the pressure member 305 comprises a mating element 310 and a pressure source 320 fluidically connected via a plurality of pressure lines to a plurality of outlets of the mating element 310. To control the pressure source 320, the pulp molding system 300A further comprises a controller 330 operatively connected to the pressure source 320. For illustrative purposes the open spaces through the mating element 310 have been represented using dashed lines.

The molded fiber toolset 350 of the pulp molding system 300A comprises holes therethrough and includes a forming screen 355 mounted onto a forming mold 351. In some examples, the forming mold 351 and the forming screen 355 may have shapes to which a wet part may be molded when formed on the forming screen 355. The forming mold 351 includes holes 352 and the forming screen 355 comprises pores 357, the holes 352 having a larger diameter than the pores 357. In some examples, the diameters or widths of the holes 352 and the pores 357 may be sized based on sizes of fibers to be used for the generation of the wet part (e.g., the diameters of the pores 357 may be smaller than the sizes of the fibers). In some other examples, the diameters or widths of the pores 357 may be sized to enable liquid to flow through the pores 357 while blocking fibers from flowing through the pores during the generation of a wet part.

As previously described the mating element 310 of the pressure member 305 is to receive the molded fiber toolset 350. In FIG. 3A, the toolset 350 and the mating element 310 contact via a surface of the forming mold 351. In some other examples, at least one element of the toolset 350 (e.g., the forming screen 355 and/or the forming mold 351) and the mating element 310 may be shaped to engage with each other. As a result of the relative position of the toolset 350 with respect to the mating element 310, a volume 303 is defined between the forming mold 351 and the mating element 310 of the pressure member 305, the volume 303 being fluidically connected to the pressure source 320 via the plurality of outlets of the mating element 310 and the plurality of pressure lines. Accordingly, upon a slurry of fibers (not shown in FIG. 3A) being in contact with the toolset 350 (e.g., via an external surface of the forming screen 355), the plurality of outlets of the mating element is fluidically connected to the liquid vehicle of the slurry of fibers via the holes of the toolset 350 (i.e., via the holes 352 in the forming mold 351 and the pores 357 in the forming screen 350). In the example represented in FIG. 3A, possible fluid pathways towards the outlets of the mating element 310 have been represented using arrows.

The controller 330 of the pulp molding system 300A is to control the pressure source 320 to separately pressurize the pressure lines. In some other examples, the controller 330 may selectively pressurize the pressure lines based on a geometry of the forming mold 351. In other examples, the controller 330 may selectively pressurize the pressure lines based on a geometry of the forming mold 351 and a geometry of the forming screen 355. In yet other examples, the controller 330 may selectively pressurize the pressure lines based on at least one of a geometry of the forming mold 351, a geometry of the forming screen 355, and a geometry of a pulp part to be generated from the wet part.

In some examples, the controller 330 of the pulp molding system 300A is to control the pressure source to selectively pressurize the pressure lines at different vacuum pressures to obtain a uniform pressure across an inner surface of the forming mold 351 facing the volume 303.

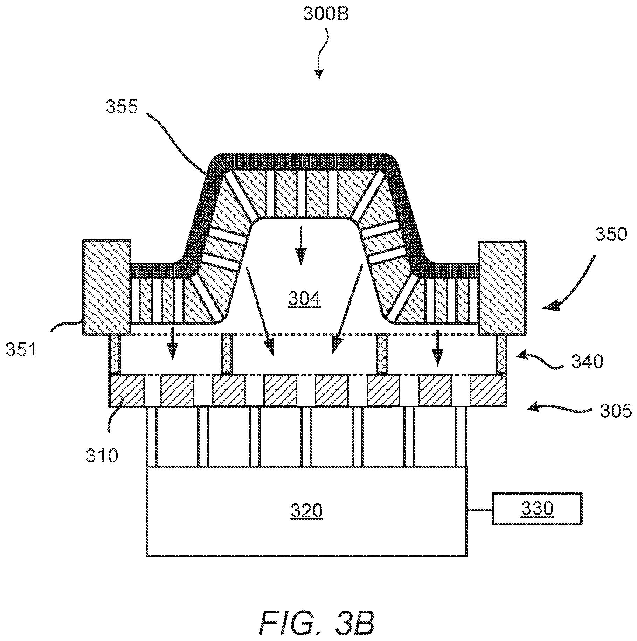

Referring now to FIG. 3B, a cross-sectional view of a pulp molding system 300B including an alignment member 340 in between a molded fiber toolset 350 and a mating element 310 of a pressure member 305 is shown. Elements previously described in reference to the pulp molding system 300A of FIG. 3A have been referenced using the same reference numerals. The dashed lines of the alignment member 340 and the mating element 310 should be interpreted as open spaces through the elements.

In the pulp molding system 300B, the alignment member 340 comprises a plurality of apertures therethrough. In the cross-sectional view of FIG. 3B, a first, a second, and a third apertures are represented. The mating element 310 comprises a plurality of outlets, each outlet being fluidically connected to a further volume 304 defined between the toolset 350 and the alignment member 340 via the apertures of the alignment member 340. Accordingly, upon a pressure source 320 of the pressure member 305 pressurizing a plurality of pressure lines fluidically connected to the plurality of outlets, a pressure value across the further volume 304 may be separately modified. As previously described, a controller 330 of the pulp molding system 300B may separately pressurize the pressure lines. In some examples, the controller 330 may control the pressure source 320 to selectively modify pressure values across the further volume 304, for instance, by controlling the pressure source 320 to selectively pressurize the pressure lines based on a geometry of the forming mold 351.

In some examples, the alignment member 340 may be used for assisting in the connection of the toolset 350 and the mating element 310 of the pressure member 305. In an example, the mating element 310 may be arranged to engage with the apertures of the alignment member 340. In some examples, as previously disclosed, the mating element 310 may comprise a plurality of manifolds, wherein each of the manifolds includes a corresponding connector to engage with at least one aperture.

Although in the pulp molding systems 300A and 300B the arrows represent a vacuum operation in which air from the volume 303 and the additional volume 304 is sucked into the pressure source 320 via the pressure lines, in other examples, the pressure source 320 may be used to blow air into the volume 303 and the further volume 304, for example to detach a wet part away from the toolset 350. However, for illustrative purposes, systems 300A and 300B have been described in reference to examples corresponding to vacuum operations.

It should be noted that, although in the pulp molding systems 300A and 300B the mating element 310 is to receive the forming mold 351, in other examples the mating element 310 may receive a transfer mold.

According to some examples, a pressure regulation device may be used for generating a second surface of the wet part and/or for transferring the wet part. In some examples, the molded fiber toolset may comprise a forming mold, a forming screen, and a transfer mold. In other examples, the molded fiber toolset may comprise a forming mold, a forming screen, a transfer mold, and a transfer screen. As previously described, a mating element of the pressure regulation device may be arranged to receive a forming mold or a transfer mold.

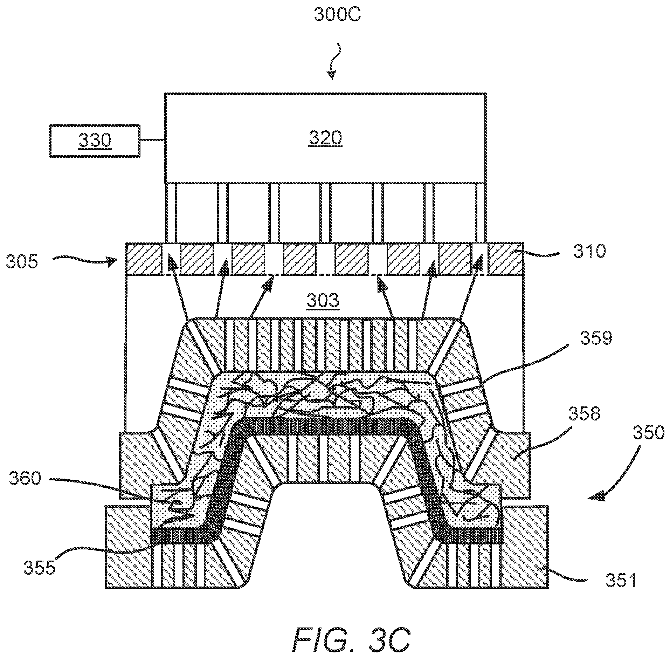

Referring now to FIG. 3C, a cross-sectional view of a pulp molding system 300C including a molded fiber toolset 350 and a wet part 360 is shown. Elements of the pulp molding system 300C previously described in reference to FIGs. 3A and 3B have been referenced using the same reference numerals. The pulp molding system 300C comprises the molded fiber toolset 350, a pressure member 305, and a controller 330. The molded fiber toolset 350 comprises a forming mold 351, a forming screen 355, and a transfer mold 358. In between the forming screen 355 and the transfer mold 358, a wet part 360 is represented. The wet part 360 may correspond to the wet part obtained in a process in which a vacuum pressure is applied to a slurry of fibers via the apertures of the molded fiber toolset 350.

The molded fiber toolset 350 may be used in at least one of a generation process in which a second surface of the wet part 360 is generated, a transfer process in which the wet part 360 is transferred from the forming screen 355 to the transfer mold 358, and a transfer process in which the wet part 360 is pulled away from the molded fiber toolset 350. In some other examples, the molded fiber toolset 350 may further comprise a transfer screen. The transfer mold 358, which comprises a plurality of holes 359 therethrough, is in contact with a mating element 310 such that a volume 303 in between the transfer mold 358 and the mating element 310 is fluidically connected to the outlets of the mating element 310. To adjust a pressure value across the volume 303, the controller 330 may control the pressure source 320 to separately pressurize the pressure lines. In some examples, the controller 330 may selectively pressurize the pressure lines based on a geometry of the transfer mold 358. In some other examples, the controller 330 may selectively pressurize the pressure lines based on a geometry of the transfer mold 358 and/or a geometry of the wet part 360 to be obtained.

When using the mating member 310, the pressure source 320, and the controller 330 to perform transfer operations, defects resulting from the application of excessive or deficient pressure levels across the wet part 360 are reduced. As previously described, excessive and deficient pressure levels may result in at least one of uneven mechanical properties across the wet part 360, portions of the wet part 360 being stuck onto the screen, presence of cracks in the wet part 360, among others.

Although the pulp molding systems 300A, 300B, and 300C have been described in reference to examples of pressure regulation devices, it should be noted that alternative pressure regulations devices such as the pressure regulation devices 100, 200A, 200B, and 200C previously explained in reference to FIGs. 1 to 2C may be possible in the pulp molding systems 300A, 300B, and 300C.

According to examples, a three-dimensional (3D) fabrication system may fabricate the molded fiber toolset 350 previously described in reference to FIGs. 3A to 3C. The 3D fabrication system may be any suitable type of additive manufacturing system. Examples of suitable additive manufacturing systems may include systems that may employ curable binder jetting onto build materials (e.g., thermally or UV curable binders), print agent jetting onto build materials (e.g., fusing and/or detailing agents), selective laser sintering, stereolithography, fused deposition modeling, etc. In a particular example, the 3D fabrication system may form molded fiber toolset 350 by binding and/or fusing build material particles together. In any of these examples, the build material particles may be any suitable type of material that may be employed in 3D fabrication processes, such as a metal, a plastic (such as a nylon), a ceramic, an alloy, and/or the like. Generally speaking, higher functionality/performance molded fiber toolsets 350 may be those with the smallest pore size to block fibers of smaller sizes. Hence, some 3D fabrication system technologies may be more suited for generating the molded fiber toolset 350 than others.

According to some examples, a method for generating a wet part from a slurry of fibers may comprise using a pressure regulation device. In some examples, a method may comprise using the pressure regulation devices previously explained in reference to FIGs. 1 to 3C to reduce the part defects resulting from application of uneven pressures across the mold.

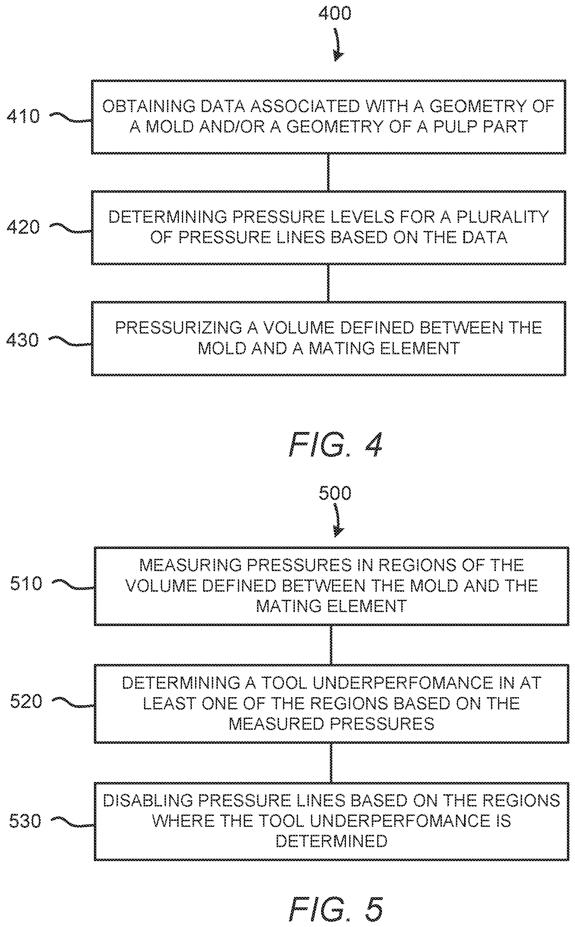

Referring now to FIG. 4, a method 400 to separately pressurize a volume defined between the mold and a mating element is shown. Method 400 may be carried out, for instance, using any of the pressure regulation devices and systems previously explained in reference to FIGs. 1 to 3B. At block 410, method 400 comprises obtaining data associated with a geometry of a mold (e.g., a forming mold or a transfer mold) and/or a geometry of a pulp part (e.g., a molded fiber part to be obtained). The data may be obtained, for instance, from a database, a memory, or as input data received from a user interface of a computing device. At block 420, method 400 comprises determining pressure levels for a plurality of pressure lines of a pressure member (e.g., a pressure member 105, 205, 305) based on the data. Then, at block 430, method 400 further comprises pressurizing a volume defined between the mold and a mating element of the pressure member via a plurality of outlets of the pressure member. Pressurizing the volume at block 430 comprises separately pressurizing the pressure lines based on the determined pressure levels. In some other examples, pressurizing the volume at block 430 may comprise selectively pressurizing the plurality of pressure lines based on the determined pressure levels.

In some examples, a pressure member for pressurizing a volume defined between a mating element and a mold in method 400 may be in fluidic communication with apertures of an alignment member (e.g., alignment member 240, 340). In an example, method 400 may further comprise engaging the mating element with an alignment member comprising a plurality of apertures therethrough, the alignment member being to receive the mold. In some examples, pressurizing the volume defined between the mold and the mating element at block 430 may comprise pressurizing a further volume defined between the mold and the alignment member via the plurality of apertures of the alignment member. In some other examples, the mating element may comprise a plurality of manifolds (e.g., manifolds 222a-222h), and method 400 may further comprise engaging each of the manifolds with at least one aperture of the alignment member such that outlets of the manifolds. As previously explained, in some examples, manifolds of the plurality of manifolds may have different sizes.

In some examples, the pressure levels to be applied by the pressure member may be determined based on an outlet arrangement for a plurality of outlets part of the mating element. In some examples, block 420 may further comprise determining the pressure levels for the plurality of pressure lines based on an outlet arrangement across a mating element including the plurality of outlets.

In some other examples, a mold may comprise a complex shape, and an arrangement of the outlets may be determined in view of a geometry of the mold. In some examples, method 400 may further comprise determining an outlet arrangement for the plurality of outlets of the mating element based on the data and distributing the plurality of outlets throughout the mating element in accordance with the determined arrangement. As previously described, the data may be associated with the geometry of the mold and/or a geometry of a pulp part. In some examples, distributing the plurality of outlets throughout the mating element may comprise determining a manifold layout based on the determined arrangement, and engaging a plurality of manifolds including outlets to the alignment member based on the manifold layout.

Referring now to FIG. 5, a method 500 for determining a tool underperformance based on pressure measurements is shown. The pressure measurements may be obtained, for instance, via a plurality of pressure sensors (e.g., pressure sensors 223a, 223b, 223c) distributed across a volume defined between the mold and a mating element (e.g., mating element 110, 210, 310). In some examples, method 400 previously explained in FIG. 4 may further comprise method 500. At block 510, method 500 comprises measuring pressure in regions of the volume defined between the mold and the mating element. Then, at block 520, method 500 comprises determining a tool underperformance in at least one of the regions based on the measured pressures. In some examples, determining the tool underperformance may comprise comparing each of the pressure measurements to a threshold pressure value and, based on the comparison, determining the tool underperformance in regions of across the volume. Then, at block 530, method 500 comprises disabling pressure lines based on the regions where the tool underperformance is determined. In some examples, the tool underperformance may be caused by the presence of elements in a fluid path (e.g., clogged apertures in a toolset including the mold, blockage of an outlet of the mating element, blockage of a pressure line). Hence, when disabling the pressure lines associated where the tool underperformance has been determined, negative defects resulting from the tool underperformance (e.g., part defects) may be prevented. In some other examples, block 530 may further comprise issuing a signal associated with the tool underperformance. In some examples, the signal may be a visual signal represented in a user interface (e.g., a display) or an acoustic signal for alerting operators of the tool underperformance.

Although the examples disclosed throughout the description have been described referring to pressure members to receive a single mold in a mating element, it should be noted that in other examples, a mating element of a pressure member (or an alignment member, if any) may receive a plurality of molds. The pressure source is capable of separately pressurizing each of the pressure lines, and hence, the use of the pressure member is not limited to a single mold. In some examples, a pressure regulation device (e.g., pressure regulation device 100, 200A, 200B, 200C) may receive a plurality of molds such that a plurality of volumes is defined in between the plurality of molds and the mating element (e.g., mating element 110, 210, 310). In an example, each of the volumes may be fluidically connected to at least two outlets of the mating member, and the controller is to control the pressure source to separately pressurize each of the pressure lines that fluidly connect the pressure source to the outlets of the mating element. In some examples, the controller may selectively pressurize each of the pressure lines in accordance with geometries of the molds to be received by the mating member. By receiving a plurality of molds in the mating element, the flexibility and the throughput of at least one of the formation process and the transportation process is enhanced compared to other pressure systems arranged to individually arrange with a mold.

What has been described and illustrated herein are examples of the invention along with some variations. The terms, descriptions, and figures used herein are set forth by way of illustration only and are not meant as limitations.

## Claims

1. A pressure regulation device (100, 200A-C) comprising:
a pressure member (105, 205, 305) comprising:
a mating element (110, 210, 310) comprising a plurality of outlets (111a-j, 211a-h), and
a pressure source (120, 220, 320) comprising a plurality of pressure lines fluidically connected to the plurality of outlets (111a-j, 211a-h); and
a controller (130, 230, 330) to control the pressure source (120, 220, 320) to separately pressurize each of the pressure lines,
wherein the mating element (110, 210, 310) is to receive a mold such that a volume defined between the mold and the mating element is fluidically connected to the plurality of outlets (111a-j, 211a-h).

2. The pressure regulation device (100, 200A-C) of Claim 1, further comprising an alignment member (240, 340) to receive the mating element (110, 210, 310), the alignment member (240, 340) comprising a plurality of apertures (241a-d) therethrough,
wherein the alignment member (240, 340) is to receive the mold such that an additional volume (303) defined between the mold and the alignment member (240, 340) is fluidically connected to the plurality of outlets (111a-j, 211a-h) via the plurality of apertures (241a-d) of the alignment member (240, 340).

3. The pressure regulation of Claim 2, wherein the alignment member (240, 340) is a lattice frame comprising the plurality of apertures (241a-d) uniformly distributed throughout the lattice frame.

4. The pressure regulation device (100, 200A-C) of Claim 2, wherein:
the mating element (110, 210, 310) comprises a plurality of manifolds (222a-h), and
each of the manifolds (222a-h) is to engage with at least one aperture of the plurality of apertures (241a-d).

5. The pressure regulation device (100, 200A-C) of Claim 4, wherein:
each aperture has a polygonal cross-section and each manifold comprises a connector having a complementary shape to engage with at least one of the apertures (241a-d).

6. The pressure regulation device (100, 200A-C) of Claim 1, wherein:
the plurality of outlets (111a-j, 211a-h) of the mating element (110, 210, 310) is distributed throughout the mating element (110, 210, 310) in an outlet arrangement, and
the controller (130, 230, 330) is to separately pressurize the pressure lines by selectively pressurizing the pressure lines based on a geometry of the mold and the outlet arrangement.

7. The pressure regulation device (100, 200A-C) of Claim 1, further comprising a plurality of sensors (223a-c) to measure pressures in regions of the volume (303),
wherein the controller (130, 230, 330) is to determine a tool underperformance based on the pressure measurements by the plurality of sensors (223a-c).

8. A method comprising:
obtaining data associated with a geometry of a mold and/or a geometry of a pulp part;
determining pressure levels for a plurality of pressure lines of a pressure member (105, 205, 305) based on the data; and
pressurizing a volume (303) defined between the mold and a mating element (110, 210, 310) of the pressure member (105, 205, 305) via a plurality of outlets (111a-j, 211a-h) of the pressure member (105, 205, 305),
wherein pressurizing the volume (303) comprises separately pressurizing the pressure lines based on the determined pressure levels.

9. The method of Claim 8, further comprising:
measuring pressures in regions of the volume (303) defined between the mold and the mating element (110, 210, 310);
determining a tool underperformance in at least one of the regions based on the measured pressures; and
disabling pressure lines based on the regions where the tool underperformance is determined.

10. The method of Claim 8, further comprising:
engaging the mating element (110, 210, 310) with an alignment member (240, 340) comprising a plurality of apertures (241a-d) therethrough, the alignment member (240, 340) to receive the mold,
wherein pressurizing the volume (303) defined between the mold and the mating element (110, 210, 310) of the pressure member (105, 205, 305) further comprises pressurizing an additional volume (303) defined between the mold and the alignment member (240, 340) via the plurality of apertures (241a-d) of the alignment member (240, 340).

11. The method of Claim 10, wherein the mating element (110, 210, 310) comprises a plurality of manifolds (222a-h), each manifold comprising an outlet of the plurality of outlets (111a-j, 211a-h), wherein engaging the mating element (110, 210, 310) with the alignment member (240, 340) comprises:
engaging each of the manifolds (222a-h) with at least one aperture of the alignment member (240, 340).

12. The method of Claim 8, further comprising:
determining an outlet arrangement for the plurality of outlets across the mating element (110, 210, 310) based on the data; and
distributing the plurality of outlets throughout the mating element (110, 210, 310) in accordance with the determined arrangement.

13. The method of Claim 8, wherein determining pressure levels for a plurality of pressure lines further comprises determining pressure levels for the plurality of pressure lines based on an outlet arrangement across the mating element (110, 210, 310).

14. A pulp molding system (300A-C) comprising:
a molded fiber toolset (350) comprising holes (352, 359) therethrough; and
a pressure member (105, 205, 305) comprising:
a mating element (110, 210, 310) to receive the molded fiber toolset (350), the mating element (110, 210, 310) comprising a plurality of outlets, and
a pressure source (120, 220, 320) fluidically connected to the plurality of outlets via a plurality of pressure lines; and
a controller (130, 230, 330) to control the pressure source (120, 220, 320) of the pressure member (105, 205, 305) to separately pressurize the pressure lines,
wherein upon a slurry of fibers being in contact with the molded fiber toolset (350), the plurality of outlets of the mating element (110, 210, 310) is fluidically connected to the slurry of fibers via the holes (352, 359) of the molded fiber toolset (350).

15. The pulp molding system (300A-C) of Claim 14, further comprising an alignment member (240, 340) in between the molded fiber toolset (350) and the mating element (110, 210, 310) of the pressure member (105, 205, 305), the alignment member (240, 340) comprising a plurality of apertures (241a-241d) therethrough, wherein upon the molded fiber toolset (350) being in contact with the slurry of fibers, the plurality of outlets is fluidically connected to the slurry of fibers via the holes (352, 359) of the molded fiber toolset (350) and the plurality of apertures (241a-241d) of the alignment member (240, 340).

## Patentansprüche

1. Druckregelvorrichtung (100, 200A-C), die umfasst:
ein Druckelement (105, 205, 305), das umfasst:
ein Gegenelement (110, 210, 310), das eine Vielzahl von Auslässen (111a-j, 211a-h) umfasst, und
eine Druckquelle (120, 220, 320), die eine Vielzahl von Druckleitungen umfasst, die fluidisch mit der Vielzahl von Auslässen (111a-j, 211a-h) verbunden sind; und
einen Controller (130, 230, 330) zum Steuern der Druckquelle (120, 220, 320), um jede der Druckleitungen getrennt mit Druck zu beaufschlagen,
wobei das Gegenelement (110, 210, 310) dazu dient, eine Form aufzunehmen, sodass ein zwischen der Form und dem Gegenelement definiertes Volumen fluidisch mit der Vielzahl von Auslässen (111a-j, 211a-h) verbunden ist.

2. Druckregelvorrichtung (100, 200A-C) nach Anspruch 1, die ferner ein Ausrichtungselement (240, 340) zur Aufnahme des Gegenelements (110, 210, 310) umfasst, wobei das Ausrichtungselement (240, 340) eine Vielzahl von dort hindurch verlaufenden Öffnungen (241 a-d) umfasst,
wobei das Ausrichtungselement (240, 340) dazu dient, die Form derart aufzunehmen, dass ein zusätzliches Volumen (303), das zwischen der Form und dem Ausrichtungselement (240, 340) definiert ist, über die Vielzahl von Öffnungen (241 a-d) des Ausrichtungselements (240, 340) fluidisch mit der Vielzahl von Auslässen (111a-j, 211a-h) verbunden ist.

3. Druckregulierung nach Anspruch 2, wobei das Ausrichtungselement (240, 340) ein Gitterrahmen ist, der die Vielzahl von Öffnungen (241a-d) umfasst, die gleichmäßig über den gesamten Gitterrahmen verteilt sind.

4. Druckregelvorrichtung (100, 200A-C) nach Anspruch 2, wobei:
das Gegenelement (110, 210, 310) eine Vielzahl von Verteilern (222a-h) umfasst, und
jeder der Verteiler (222a-h) dazu dient, mit mindestens einer Öffnung der Vielzahl von Öffnungen (241 a-d) in Eingriff zu stehen.

5. Druckregelvorrichtung (100, 200A-C) nach Anspruch 4, wobei:
jede Öffnung einen polygonalen Querschnitt aufweist und jeder Verteiler einen Verbinder mit einer komplementären Form zum Eingriff in mindestens eine der Öffnungen (241 a-d) umfasst.

6. Druckregelvorrichtung (100, 200A-C) nach Anspruch 1, wobei:
die Vielzahl von Auslässen (111a-j, 211a-h) des Gegenelements (110, 210, 310) über das gesamte Gegenelement (110, 210, 310) in einer Auslassanordnung verteilt ist, und
der Controller (130, 230, 330) dazu dient, die Druckleitungen getrennt mit Druck zu beaufschlagen, indem sie die Druckleitungen basierend auf einer Geometrie der Form und der Auslassanordnung selektiv mit Druck beaufschlagt.

7. Druckregelvorrichtung (100, 200A-C) nach Anspruch 1, die ferner eine Vielzahl von Sensoren (223a-c) zum Messen von Drücken in Bereichen des Volumens (303) umfasst,
wobei der Controller (130, 230, 330) dazu dient, basierend auf den Druckmessungen durch die Vielzahl von Sensoren (223a-c) eine unzureichende Werkzeugleistung zu bestimmen.

8. Verfahren, das umfasst:
Erhalten von Daten, die mit einer Geometrie einer Form und/oder einer Geometrie eines Faserformteils verknüpft sind;
Bestimmen von Druckniveaus für eine Vielzahl von Druckleitungen eines Druckelements (105, 205, 305) basierend auf den Daten; und Druckbeaufschlagen eines Volumens (303), das zwischen der Form und einem Gegenelement (110, 210, 310) des Druckelements (105, 205, 305) definiert ist, über eine Vielzahl von Auslässen (111a-j, 211a-h) des Druckelements (105, 205, 305),
wobei das Druckbeaufschlagen des Volumens (303) das getrennte Druckbeaufschlagen der Druckleitungen basierend auf den bestimmten Druckniveaus umfasst.

9. Verfahren nach Anspruch 8, das ferner umfasst:
Messen von Drücken in Bereichen des zwischen der Form und dem Gegenelement (110, 210, 310) definierten Volumens (303);
Bestimmen einer unzureichenden Werkzeugleistung in mindestens einem der Bereiche basierend auf den gemessenen Drücken; und
Deaktivieren von Druckleitungen basierend auf den Bereichen, in denen die unzureichende Werkzeugleistung bestimmt wird.

10. Verfahren nach Anspruch 8, das ferner umfasst:
In-Eingriff-Bringen des Gegenelements (110, 210, 310) mit einem Ausrichtungselement (240, 340), das eine Vielzahl von Durchgangsöffnungen (241a-d) umfasst, wobei das Ausrichtungselement (240, 340) zur Aufnahme der Form dient,
wobei das Druckbeaufschlagen des Volumens (303), das zwischen der Form und dem Gegenelement (110, 210, 310) des Druckelements (105, 205, 305) definiert ist, ferner das Druckbeaufschlagen eines zusätzlichen Volumens (303), das zwischen der Form und dem Ausrichtungselement (240, 340) definiert ist, über die Vielzahl von Öffnungen (241 a-d) des Ausrichtungselements (240, 340) umfasst.

11. Verfahren nach Anspruch 10, wobei das Gegenelement (110, 210, 310) eine Vielzahl von Verteilern (222a-h) umfasst, wobei jeder Verteiler einen Auslass der Vielzahl von Auslässen (111a-j, 211a-h) umfasst, wobei das In-Eingriff-Bringen des Gegenelements (110, 210, 310) mit dem Ausrichtungselement (240, 340) umfasst:
In-Eingriff-Bringen eines jeden der Verteiler (222a-h) mit mindestens einer Öffnung des Ausrichtungselements (240, 340).

12. Verfahren nach Anspruch 8, das ferner umfasst:
Bestimmen einer Auslassanordnung für die Vielzahl von Auslässen über das Verbindungselement (110, 210, 310) basierend auf den Daten; und
Verteilen der Vielzahl von Auslässen über das gesamte Gegenelement (110, 210, 310) gemäß der bestimmten Anordnung.

13. Verfahren nach Anspruch 8, wobei das Bestimmen von Druckniveaus für eine Vielzahl von Druckleitungen ferner das Bestimmen von Druckniveaus für die Vielzahl von Druckleitungen basierend auf einer Auslassanordnung über das Gegenelement (110, 210, 310) umfasst.

14. Faserformsystem (300A-C), das umfasst:
einen geformten Faserwerkzeugsatz (350), der dort hindurch verlaufende Löcher (352, 359) umfasst; und
ein Druckelement (105, 205, 305), das umfasst:
ein Gegenelement (110, 210, 310) zur Aufnahme des geformten Faserwerkzeugsatzes (350), wobei das Gegenelement (110, 210, 310) eine Vielzahl von Auslässen umfasst, und
eine Druckquelle (120, 220, 320), die über eine Vielzahl von Druckleitungen fluidisch mit der Vielzahl von Auslässen verbunden ist; und
einen Controller (130, 230, 330) zum Steuern der Druckquelle (120, 220, 320) des Druckelements (105, 205, 305), um die Druckleitungen getrennt mit Druck zu beaufschlagen,
wobei bei Kontakt einer Aufschlämmung von Fasern mit dem Faserformwerkzeugsatz (350) die Vielzahl von Auslässen des Gegenelements (110, 210, 310) über die Löcher (352, 359) des Faserformwerkzeugsatzes (350) fluidisch mit der Aufschlämmung von Fasern verbunden ist.

15. Faserformsystem (300A-C) nach Anspruch 14, das ferner ein Ausrichtungselement (240, 340) zwischen dem Formfaserwerkzeugsatz (350) und dem Gegenelement (110, 210, 310) des Druckelements (105, 205, 305) umfasst, wobei das Ausrichtungselement (240, 340) eine Vielzahl von dort hindurch verlaufende Öffnungen (241a-241d) umfasst, wobei, wenn der Formfaserwerkzeugsatz (350) in Kontakt mit der Aufschlämmung von Fasern ist, die Vielzahl von Auslässen über die Löcher (352, 359) des Formfaserwerkzeugsatzes (350) und die Vielzahl von Öffnungen (241a-241d) des Ausrichtungselements (240, 340) fluidisch mit der Aufschlämmung von Fasern verbunden ist.

## Revendications

1. Dispositif de régulation de pression (100, 200A-C) comprenant :
un élément de pression (105, 205, 305), comprenant :
un élément homologue (110, 210, 310) comprenant une pluralité de sorties (111a-j, 211a-h), et
une source de pression (120, 220, 320) comprenant une pluralité de conduites de pression reliées fluidiquement à la pluralité de sorties (111a-j, 211a-h) ; et
un dispositif de commande (130, 230, 330) pour commander la source de pression (120, 220, 320) afin de pressuriser séparément chacune des conduites de pression,
dans lequel l'élément d'accouplement (110, 210, 310) doit recevoir un moule de telle sorte qu'un volume défini entre le moule et l'élément d'accouplement est relié fluidiquement à la pluralité de sorties (111a-j, 211a-h).

2. Dispositif de régulation de pression (100, 200A-C) selon la revendication 1, comprenant en outre un élément d'alignement (240, 340) pour recevoir l'élément d'accouplement (110, 210, 310), l'élément d'alignement (240, 340) comprenant une pluralité d'ouvertures (241 a-d) à travers celui-ci,
dans lequel l'élément d'alignement (240, 340) doit recevoir le moule de telle sorte qu'un volume supplémentaire (303) défini entre le moule et l'élément d'alignement (240, 340) est relié fluidiquement à la pluralité de sorties (111a-j, 211a-h) par l'intermédiaire de la pluralité d'ouvertures (241 a-d) de l'élément d'alignement (240, 340).

3. Régulation de pression selon la revendication 2, dans laquelle l'élément d'alignement (240, 340) est un cadre en treillis comprenant la pluralité d'ouvertures (241 a-d) uniformément réparties sur l'ensemble du cadre en treillis.

4. Dispositif de régulation de pression (100, 200A-C) selon la revendication 2, dans lequel :
l'élément d'accouplement (110, 210, 310) comprend une pluralité de collecteurs (222a-h), et
chacun des collecteurs (222 a-h) doit se mettre en prise avec au moins une ouverture de la pluralité d'ouvertures (241 a-d).

5. Dispositif de régulation de pression (100, 200A-C) selon la revendication 4, dans lequel :
chaque ouverture a une section transversale polygonale et chaque collecteur comprend un connecteur ayant une forme complémentaire pour venir en prise avec au moins l'une des ouvertures (241 a-d).

6. Dispositif de régulation de pression (100, 200A-C) selon la revendication 1, dans lequel :
la pluralité de sorties (111a-j, 211a-h) de l'élément homologue (110, 210, 310) est répartie dans l'ensemble de l'élément homologue (110, 210, 310) dans un agencement de sortie, et
le dispositif de commande (130, 230, 330) doit mettre sous pression séparément les conduites de pression en mettant sélectivement sous pression les conduites de pression sur la base d'une géométrie du moule et de l'agencement de sortie.

7. Dispositif de régulation de pression (100, 200A-C) selon la revendication 1, comprenant en outre une pluralité de capteurs (223 a-c) pour mesurer des pressions dans des régions du volume (303),
dans lequel le dispositif de commande (130, 230, 330) doit déterminer une sous-performance d'outil sur la base des mesures de pression par la pluralité de capteurs (223 a-c).

8. Procédé comprenant :
l'obtention de données associées à une géométrie d'un moule et/ou à une géométrie d'une pièce de pâte à papier ;
la détermination de niveaux de pression pour une pluralité de conduites de pression d'un élément de pression (105, 205, 305) sur la base des données ; et la mise sous pression d'un volume (303) défini entre le moule et un élément d'accouplement (110, 210, 310) de l'élément de pression (105, 205, 305) par l'intermédiaire d'une pluralité de sorties (111a-j, 211a-h) de l'élément de pression (105, 205, 305),
dans lequel la pressurisation du volume (303) comprend la pressurisation séparée des conduites de pression sur la base des niveaux de pression déterminés.

9. Procédé selon la revendication 8, comprenant en outre :
la mesure de pressions dans les régions du volume (303) définies entre le moule et l'élément d'accouplement (110, 210, 310) ;
la détermination d'une sous-performance d'outil dans au moins une des régions sur la base des pressions mesurées ; et
la désactivation des conduites de pression en fonction des régions où la sous-performance de l'outil est déterminée.

10. Procédé selon la revendication 8, comprenant en outre :
la mise en prise de l'élément homologue (110, 210, 310) avec un élément d'alignement (240, 340) comprenant une pluralité d'ouvertures (241a-d) à travers celui-ci, l'élément d'alignement (240, 340) devant recevoir le moule,
dans lequel la mise sous pression du volume (303) défini entre le moule et l'élément d'accouplement (110, 210, 310) de l'élément de pression (105, 205, 305) comprend en outre la mise sous pression d'un volume supplémentaire (303) défini entre le moule et l'élément d'alignement (240, 340) par l'intermédiaire de la pluralité d'ouvertures (241 a-d) de l'élément d'alignement (240, 340).

11. Procédé selon la revendication 10, dans lequel l'élément d'accouplement (110, 210, 310) comprend une pluralité de collecteurs (222a-h), chaque collecteur comprenant une sortie de la pluralité de sorties (111a-j, 211a-h), dans lequel la mise en prise de l'élément d'accouplement (110, 210, 310) avec l'élément d'alignement (240, 340) comprend :
la mise en prise de chacun des collecteurs (222a-h) avec au moins une ouverture de l'élément d'alignement (240, 340).

12. Procédé selon la revendication 8, comprenant en outre :
la détermination d'un agencement de sortie pour la pluralité de sorties à travers l'élément homologue (110, 210, 310) sur la base des données ; et
la distribution de la pluralité de sorties au travers de l'élément homologue (110, 210, 310) conformément à l'agencement déterminé.

13. Procédé selon la revendication 8, dans lequel la détermination des niveaux de pression pour une pluralité de conduites de pression comprend en outre la détermination de niveaux de pression pour la pluralité de conduites de pression sur la base d'un agencement de sortie à travers l'élément homologue (110, 210, 310).

14. Système de moulage de pâte (300A-C) comprenant :
un jeu d'outils en fibre moulée (350) comprenant des trous (352, 359) à travers celui-ci ; et
un élément de pression (105, 205, 305), comprenant :
un élément d'accouplement (110, 210, 310) destiné à recevoir l'ensemble d'outils en fibre moulée (350), l'élément d'accouplement (110, 210, 310) comprenant une pluralité de sorties, et
une source de pression (120, 220, 320) reliée fluidiquement à la pluralité de sorties par l'intermédiaire d'une pluralité de conduites de pression ; et
un dispositif de commande (130, 230, 330) pour commander la source de pression (120, 220, 320) de l'élément de pression (105, 205, 305) pour pressuriser séparément les conduites de pression,
dans lequel, lorsqu'une suspension de fibres est en contact avec l'ensemble d'outils en fibre moulée (350), la pluralité de sorties de l'élément d'accouplement (110, 210, 310) est reliée fluidiquement à la suspension de fibres par l'intermédiaire des trous (352, 359) de l'ensemble d'outils en fibre moulée (350).

15. Système de moulage de pâte à papier (300A-C) selon la revendication 14, comprenant en outre un élément d'alignement (240, 340) entre l'ensemble d'outils en fibre moulée (350) et l'élément d'accouplement (110, 210, 310) de l'élément de pression (105, 205, 305), l'élément d'alignement (240, 340) comprenant une pluralité d'ouvertures (241a-241d) à travers celui-ci, dans lequel, lorsque l'ensemble d'outils en fibre moulée (350) étant en contact avec la suspension de fibres, la pluralité de sorties est reliée fluidiquement à la suspension de fibres par l'intermédiaire des trous (352, 359) de l'ensemble d'outils en fibre moulée (350) et la pluralité d'ouvertures (241a-241d) de l'élément d'alignement (240, 340).
